(19) **Europäisches Patentamt**

**European Patent Office**

**Office européen des brevets**

(11) **EP 0 631 138 B1**

(12) **EUROPEAN PATENT SPECIFICATION**

(45) Date of publication and mention
of the grant of the patent:
**22.11.2000 Bulletin 2000/47**

(51) Int. Cl.[7]: **G01N 33/92**, G01N 24/08,
C12Q 1/26

(21) Application number: **93111276.7**

(22) Date of filing: **14.07.1993**

(54) **Process for detecting and determining oxidized lipid**

Verfahren zum Nachweis und zur Bestimmung von oxidierten Lipiden

Procédé pour détecter et déterminer des lipides oxidés

(84) Designated Contracting States:
**BE DE FR GB NL**

(30) Priority: **12.05.1993 JP 14417093**

(43) Date of publication of application:
**28.12.1994 Bulletin 1994/52**

(73) Proprietor: **Koike, Katsumasa**
**Tokyo 162 (JP)**

(72) Inventor: **Koike, Katsumasa**
**Tokyo 162 (JP)**

(74) Representative:
**Bühling, Gerhard, Dipl.-Chem.**
**Patentanwaltsbüro**
**Tiedtke-Bühling-Kinne & Partner**
**Bavariaring 4**
**80336 München (DE)**

(56) References cited:
**EP-A- 0 141 343**       **WO-A-93/03450**
**DE-A- 3 809 171**       **US-A- 4 206 132**
**US-A- 4 677 247**

• CHEMICAL ABSTRACTS, vol. 112, no. 19, 7 May
1990, Columbus, Ohio, US; abstract no. 175109a,
BARENGHI, L. ET AL 'NMR analysis of low-
density lipoprotein oxidatively -modified in vitro'
& FREE RADICAL RES. COMMUN., 8(3), 175-83
1990

• DATABASE WPI Section Ch, Week 8520, Derwent
Publications Ltd., London, GB; Class E12, AN
85-118817 'Fluorine-containg. optical analytical
material - comprises lanthanide chelate of
fluoro-beta-diketone and metalsalt of fatty acid'
& JP-A-60 058 538 (DAIKIN KOGYO KK) 4 April
1985

• AGRICULTURAL AND BIOLOGICAL CHEMISTRY
vol. 52, no. 3 , March 1988 , TOKYO JP pages 849
- 850 RYO YAMAUCHI ET AL. 'Effect of Metal
Salts and Fructose on the Autoxidation of Methyl
Linoleate in Emulsions'

• CHEMICAL ABSTRACTS, vol. 115, no. 17, 28
October 1991, Columbus, Ohio, US; abstract no.
178256u, PUNNONEN, K. ET AL 'Effects of
ultraviolet A and B irradiation on lipid
peroxidation and activity of the antioxidant
enzymes in keratinocytes in culture' &
PHOTODERMATOL. PHOTOIMMUNOL.
PHOTOMED., 8(1), 3-6 1991

• CHEMICAL ABSTRACTS, vol. 109, no. 25, 19
December 1988, Columbus, Ohio, US; abstract
no. 225930x, HIRAMITSU, TADAHISA ET AL
'Effects of superoxide dismutase and catalase
on UV -light induced retina lipid peroxidation' &
ATARASHII GANKA, 5(7), 1024-6 1988

EP 0 631 138 B1

- ANALYTICAL BIOCHEMISTRY vol. 193, no. 2 , March 1991 , NEW YORK US pages 204 - 211 GREGORY L. CRAMER ET AL. 'Iodimetric Measurement of Lipid Hydroperoxides in Human Plasma'
- Methods of Enzymatic Analysis, Bergmeyer, vol. II, 3rd edition, 1983, VCH Verlag, Weinheim, Germany
- Römpp Chemie-Lexikon, 9th edition, 1990, p. 1011
- Römpp Chemie-Lexikon, 9th edition, 1992, p. 4393-4394

## Description

## BACKGROUND OF THE INVENTION

The present invention relates to a process for detecting and determining a water-soluble oxidized lipid, which is capable of readily confirming a specimen to contain a water-soluble oxidized lipid.

Oxidized lipids are believed to cause adverse effects *in vivo*, and are pointed out to be responsible for arteriosclerosis, cancer, inflammation and the like. It is also believed that low density lipoproteins which account for a majority of lipids in blood are greatly related to the above-mentioned disorders.

On the other hand, oxidized lipids which are controlled and produced by enzymes *in vivo*, such as prostaglandin and leukotriene are likely to become substances having a strong influence on a living body. Various methods for detecting a stable oxidized lipid or a final metabolite have been developed. These methods include, for example, iodine oxidation method which comprises ionizing iodine by oxidation, followed by emission spectroscopic analysis on the ionized iodine; TBARS (thiobarbituric acid reactive substance) measuring method which comprises producing a pigment by reaction with malondialdehyde as a peroxide lipid metabolite, followed by determination of the pigment by means of an absorption spectrophotometer or a fluorophtometer; conjugated diene determination method which comprises determining a conjugated diene based on the fact that a conjugated diene is formed by peroxidation of an unsaturated fatty acid; high performance liquid chromatography (HPLC) method which comprises separating and determining a peroxide lipid sample by means of a high performance liquid chromatograph; gas chromatography method which comprises esterifying a peroxide lipid sample, followed by separation and determination by means of a gas chromatography.

On the other hand, various methods for producing an oxidized lipid have heretofore been reported. As these methods, there may be mentioned, for example, a method which comprises adding a transition metal such as copper cation ($Cu^+$) or iron cation ($Fe^{2+}$) to a lipid sample, and allowing the mixture to stand at a temperature of about 37°C for a period of 20 to 30 hours; a method which comprises irradiating a sample that is preliminarily added with a photosensitive substance with light; a method which comprises oxidation by an enzyme such as lipoxygenase or cyclooxygenase.

However, the iodine oxidation method and the conjugated diene determination method which determine an oxidized lipid have a drawback in that they are not suitable for analysis of a sample containing an impurity. Further, the iodine oxidation method, the TBARS method, and the conjugated diene determination method are non-specific determination methods, i.e., these methods do not directly determine an oxidized lipid per se, but indirectly determine an oxidized lipid by determining a by-product which is formed through formation or decomposition of an oxidized lipid.

On the other hand, the HPLC method and the gas chromatography method are specific methods which directly determine an oxidized lipid per se. In the HPLC method, however, it takes a long period of time to complete determination, so that a sample can undergo denaturation.

In addition, an oxidized lipid per se is likely to undergo alteration by a solvent such as ethyl ether or ethanol. Further, in the gas chromatography method, a sample is likely to undergo alteration through esterification treatment or the like.

US-A-4,677,247 discloses a new variety of odorless soybean which contains a large quantity of the enzyme lipoxygenase which accelerates oxidation of unsaturated fatty acids such as linolic and linolenic acids which are components of soybean oil.

Chemical Abstracts, vol. 112, 1990, p. 382, Abstract no. 175109a relates to the NMR analysis of low-density lipoprotein oxidatively modified in vitro. These lipoproteins which have been oxidized in vitro were analyzed by $^1$H NMR spectroscopy and by biochemical methods.

US-A-4,206,132 indicates the general usefulness of lanthanide chelates having a fluorinated ligand, e.g. tris(trifluoroacetyl-d-camphorato)europium (III) which are used as nuclear magnetic resonance shift reagents for spectral simplification and clarification. As an application example the use in detecting and analyzing pollutants is indicated.

As described above, there is still demand for a method for precisely detecting and analyzing an oxidized lipid having physiological influence or an unstable oxidized lipid which adversely affects a body.

In addition, there is further demand for developing a method during which a water-soluble oxidized lipid is formed having a hydroperoxide group which has specific influence on a living body. Further, a method for precisely determining a water-soluble oxidized lipid having a hydroperoxide group has not been developed. Accordingly, it has been impossible to evaluate formation of a specific water-soluble oxidized lipid.

The present invention has been made to solve the above-mentioned problems.

## SUMMARY OF THE INVENTION

It is, therefore, an object of the present invention to provide a process for detecting and determining an oxidized lipid which is capable of readily and precisely determining a specimen to be oxidized lipid.

It is another object of the present invention to provide a process during which a water-soluble oxidized lipid is formed having a hydroperoxide group which has specific influence on a living body.

It is still another object of the present invention to provide a process which is capable of directly determining an oxidized lipid in a biological sample such as plasma.

To attain the above-mentioned objects, the present invention confirms and determines a specimen to be an oxidized lipid by adding a lanthanide shift reagent to a specimen and subjecting the resultant mixture to spectroscopy to detect and determine the water-soluble oxidized lipid.

As the specimen, linoleic acid or arachidonic acid which is oxidized by soybean lipoxygenase may be used.

As the lanthanide shift reagent, dysprosium cation may be used.

On the other hand, in the process according to the present invention, the water-soluble oxidized lipid is preferably obtainable by, addition of superoxide dismutase (SOD) and $CuSO_4$ to a linoleic acid or arachidonic acid emulsion prepared by dissolving linoleic acid or arachidonic acid in deuterated methyl alcohol and adding the solution to a deuterated phosphate buffer under stirring, or to a dissolving low density lipoprotein solution sufficiently dialyzed against undeuterated phosphate buffer, and irradiation with a long-wave ultraviolet light to form said water-soluble oxidized lipid.

Further, a specimen is detected and determined to contain an oxidized lipid by:

adding dysprosium tripolyphosphate anion to the above-described irradiated mixture containing the water-soluble oxidized lipid; and subjecting the mixture to proton NMR spectroscopy by means of a nuclear magnetic resonance spectrometer.

The present invention is directed to confirm and determine a specimen to be an oxidized lipid by adding a lanthanide shift reagent to a specimen, followed by spectroscopy on the mixture. This is based on the following fact. When a lanthanide shift reagent is added to a specimen, difference in proton signals is observed between the case where the substance is an oxidized lipid and the case where the substance is not an oxidized lipid. Accordingly, by detecting the difference through spectroscopy, the specimen is detected and determined to be an oxidized lipid.

Accordingly, the present invention is capable of detecting and determining a water-soluble oxidized lipid having a hydroperoxide group which has considerable influence on a living body by the use of a low density lipoprotein or fatty acid.

## BRIEF DESCRIPTION OF THE DRAWINGS

Fig. 1 is a graph showing the influence of dysprosium (Dy) concentration upon proton signals of an oxidized lipid;

Fig. 2 is a graph showing the change in proton peaks of oxidized linoleic acid caused by addition of glutathione and glutathione peroxidase;

Fig. 3 is a graph showing the change with time in oxidation of linoleic acid with soybean lipoxygenase;

Fig. 4 is a graph showing the change with time in oxidation of linoleic acid with soybean lipoxygenase in the presence of superoxide dismutase (SOD);

Fig. 5 is a graph showing the change with time in oxidation of arachidonic acid with soybean lipoxygenase.

Fig. 6 is a graph showing the change with time in oxidation of arachidonic acid with soybean lipoxygenase in the presence of superoxide dismutase (SOD);

Fig. 7 is a graph showing the results of measurement with time of oxidized samples by means of a proton nuclear magnetic resonance spectrometer, the oxidized samples having been prepared by preliminarily adding superoxide dismutase (SOD) and $5\mu M$ $CuSO_4$ to the linoleic acid samples, followed by irradiation with long-wave ultraviolet light;

Fig. 8 is a graph showing the results of measurement with time by means of a proton nuclear magnetic spectrometer with respect to linoleic acid samples oxidized by irradiation with long-wave ultraviolet light;

Fig. 9 is a graph showing the results of measurement of samples in such a procedure that superoxide dismutase (SOD) and $5\mu M$ $CuSO_4$ are added to a low density lipoprotein solution, then the mixture is irradiated with long-wave ultraviolet light to oxidize the low density lipoprotein, and a dysprosium tripolyphosphate solution is added thereto, followed by measurement of the resultant samples by means of a proton nuclear magnetic resonance spectrometer;

Fig. 10 is a graph showing the results of rough quantitative determination by measuring the heights of peak 1 influenced by a hydroperoxide group in low density lipoprotein; and

Fig. 11 is a graph showing the results of measurements of change with time of oxidation of the low density lipoprotein irradiated with long-wave ultraviolet light by thiobarbituric acid reaction substance measuring method and by $^1$H-NMR spectroscopy.

## DETAILED DESCRIPTION OF THE PREFERRED EMBODIMENTS

According to the present invention, a specimen is detected and determined to be an oxidized lipid by adding a lanthanide shift reagent to the specimen, followed by spectroscopy on the mixture. As the specimen, use is preferably made of linoleic acid or arachidonic acid which is each oxidized by soybean lipoxygenase. As the lanthanide shift reagent, dysprosium tripolyphosphate anion is preferably used.

In this connection, there may be used as the lanthanide shift reagent europium cation ($Eu^{3+}$) and thulium cation ($Tm^{3+}$) as well as dysprosium cation. The use of europium cation or thulium cation enables substantially the same result as in the case of dysprosium cation to be obtained. As the chelating agent, used according to claim 7 ethylenediaminetetraacetic acid and the like may be used as well as the tripolyphosphate mentioned above.

$^{13}$C-NMR spectroscopy analysis would also provide as significant information on the oxidized lipid as $^{1}$H-NMR analysis, using the lanthanide shift reagents.

Method for Oxidation of Linoleic Acid and Arachidonic Acid with Soybean Lipoxygenase

Linoleic acid and arachidonic acid are used in the present embodiment of the present invention as substances to be examined and are prepared in the following manner.

First, linoleic acid and arachidonic acid are separately dissolved into deuterated methyl alcohol (Methyl-$d_3$ alcohol-d: $CD_3OD$) at a concentration of 0.1M. The solutions are each emulsified in a 20mM oxygen-saturated deuterated phosphate buffer (pH 7.0) to prepare a 4mM linoleic acid emulsion and a 2mM arachidonic acid emulsion. Then, from the emulsions, those contain no superoxide dismutase (SOD) and those added with superoxide dismutase (SOD) at a concentration of 0.1 U/ml. For oxidation of the fatty acids, soybean lipoxygenase is added to each of the emulsions at a final concentration of 2000 U/ml. The final volume is adjusted to 1ml and oxidation is conducted at a temperature of 0°C.

After elapse of predetermined period of time, soybean lipoxygenase is inactivated by acidifying the reaction solution to pH 4 with deuterium chloride. Then, ethylenediaminetetraacetic acid is added to the solution at a concentration of 1mM, and the solution is adjusted to pH 7.0 with NaOH dissolved in deuterium oxide. The volume of the solution is finally adjusted to 1.5ml with 20mM deuterated phosphate buffer.

To the oxidized lipid samples, glutathione is added at various concentrations and glutathione peroxidase at a concentration of 0.2 U/ml, thereby adjusting the final volume to 1.5ml. Reaction is advanced at 30°C for 10 min. Thereafter, to each of the samples is added dysprosium tripolyphosphate anion as a lanthanide shift reagent at a concentration of 40mM.

In the process for forming dysprosium tripolyphosphate anion solution, 200mM dysprosium chloride ($DyCl_3$) and 480mM pentasodium tripolyphosphate are thoroughly stirred in deuterium oxide. The solution, though it has a sediment, is adjusted to pH 7.4 with deuterium chloride, thereby some of the precipitate will be dissolved. Then, the solution is centrifuged at 3000g for 30 min to remove the remaining sediment. This solution is used as 200mM dysprosium tripolyphosphate anion ($Dy(ppp)_2^{-7}$) solution which are to be added to the specimen.

Determination of Oxidized Lipid by $^{1}$H-NMR spectroscopy

According to the present invention, a specimen is detected and determined to be an oxidized lipid by adding the 200mM dysprosium tripolyphosphate anion solution as a lanthanide shift reagent to the specimen, followed by spectroscopy of the mixture. For the spectroscopy, hydrogen nucleus (proton) NMR analysis ($^{1}$H-NMR spectroscopy) by means of a 360MHz-nuclear magnetic resonance spectrometer equipped with a computerized analyzer is used.

Proton spectra are obtained at a probe temperature of 35°C, and the spectra are accumulated by repeating Free Induction Decay (FID) 512 times. $H_2O$ peak is presaturated by 4 sec. of 200mW of gated, continuous wave, single frequency radio wave. Parameters of the spectra are 8192 data points, a spectral width of 7246 Hz, and acquisition time of 0.565 sec. The spinning rate of the sample tube is 15 Hz and the frequency was deuterium-locked prior to determination.

Influence of Dysprosium Cation ($Dy^{3+}$) Concentration on Proton Signal of Oxidized Lipid

According to this embodiment of the present invention, a specimen is detected and determined to be a water-soluble oxidized lipid in view of the fact that when dysprosium cation ($Dy^{3+}$) is added to oxidized lipid samples at various concentrations, a difference in proton signals is observed between the case where the sample is a water-soluble oxidized lipid and the case where the sample is not a water-soluble oxidized lipid.

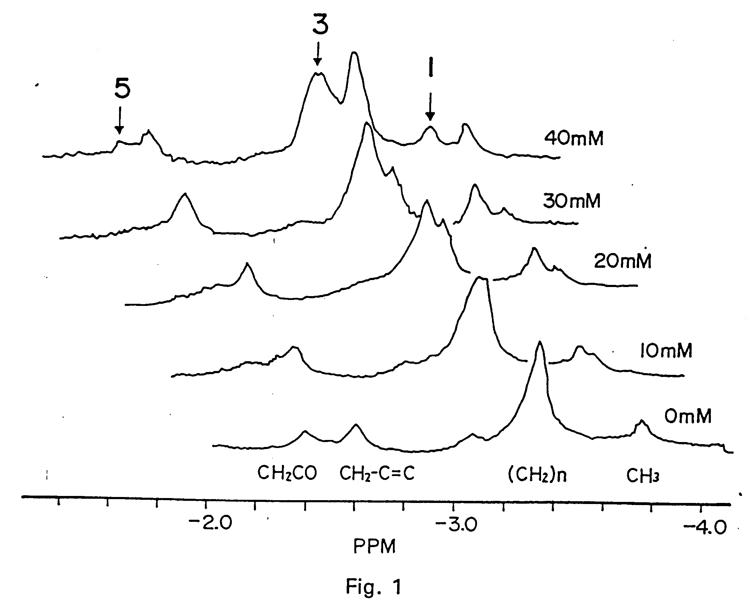

Accordingly, influence of dysprosium cation ($Dy^{3+}$) cation concentrations on proton signal of oxidized lipid is observed by adding dysprosium tripolyphosphate anion ($Dy(ppp)_2^{-7}$) to oxidized lipid samples at various concentrations (see Fig. 1).

The values in Fig. 1 show concentrations (mM) of dysprosium cation ($Dy^{3+}$) added to the samples. $CH_3$, $(CH_2)_n$, $CH_2$-C=C, and $CH_2CO$ in this Figure show proton signals in the fatty acid molecule which have already been identified, i.e., proton signals at the above-mentioned sites in the fatty acid.

The scale of the shift is based on $H_2O$ peak as the reference point (0 PPM). Peak 1 is a peak separated from $CH_3$ peak by influence of dysprosium cation ($Dy^{3+}$). This peak is verified to be a proton signal influenced by a hydroperoxide group. Peak 3 is also considered to be a proton signal influenced by a hydroperoxide group. However, it is not clearly understood to what peak 5 is due.

Change in Proton Peak of Oxidized Lipid Due to Reaction with Glutathione and Glutathione Peroxidase

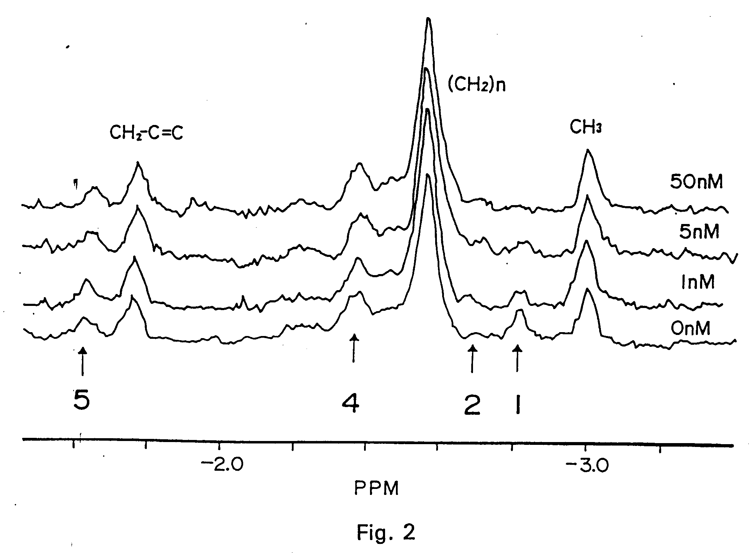

Next, using samples to which glutathione is added at various concentrations and glutathione peroxidase is added as a catalyst for reduction, the influence on proton peaks of oxidized lipid is observed (see Fig. 2).

The values in Fig. 2 show concentrations in nM of glutathione added to the samples. In this Fig. 2, it is observed that peak 1 in each of spectra becomes lowered with higher concentration of glutathione. From this, it is understood that peak 1 is a proton signal influenced by a hydroperoxide group. In contrast to peak 1, peak 2 is more distinctly observed due to the reaction with glutathion. In other words, it is considered that this peak is influenced by a hydroxide group formed by reduction of hydroperoxide group with glutathione.

Peaks 4 and 5 are not influenced by glutathione, and their derivations are not clearly understood. However, since they are observed due to oxidation of linoleic acid with lipoxygenase, they are considered to be proton signals influenced by an oxidative group such as endoperoxide.

Change with time in Oxidation of Linoleic Acid with Soybean Lipoxygenase

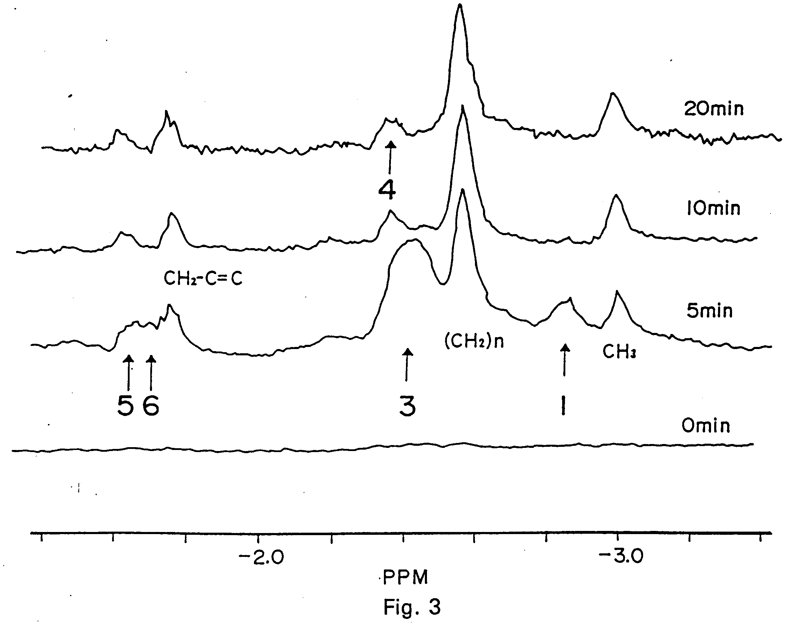

In this embodiment of the present invention, course of oxidation of linoleic acid with soybean lipoxygenase is measured by $^1$H-NMR spectroscopy (see Fig. 3).

The periods of time (min) shown in Fig. 3 indicate those for reaction with soybean lipoxygenase. Any peak is not observed in the state where linoleic acid is not oxidized with lipoxygenase, because linoleic acid is insoluble in water and hence linoleic acid is not influenced by dysprosium cation ($Dy^{3+}$). In contrast to this, linoleic acid becomes water-soluble when oxidized and oxidized lipid is influenced by dysprosium cation ($Dy^{3+}$), so that there is observed substantial difference in proton resonance frequencies between unoxidized linoleic acid and oxidized linoleic acid.

Accordingly, when the window of the spectrum is adjusted to the proton signal of the oxidized linoleic acid, the proton signal of unoxidized linoleic acid is out of the window. Consequently, the present invention is proved to be effective as a method for confirming and determining a specimen to be an oxidized lipid.

Change with Time in Oxidation of Linoleic Acid with Soybean Lipoxygenase in the Presence of Superoxide Dismutase (SOD)

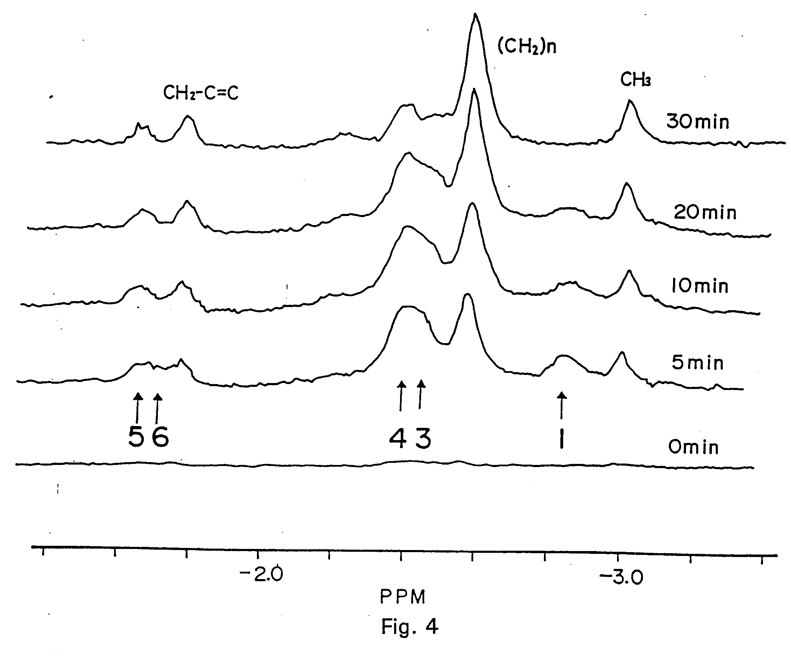

Linoleic acid is oxidized with soybean lipoxygenase in the same conditions as above except that the oxidation is conducted in the presence of 0.1 U/ml superoxide dismutase (SOD). The samples are measured with time by $^1$H-NMR spectroscopy (see Fig. 4).

The samples in the presence of superoxide dismutase (SOD) (see Fig. 4) show patterns distinctly different from those shown in the case in the absence of superoxide dismutase (SOD) (see Fig. 3). In other words, when superoxide dismutase (SOD) is present, peaks 1 and 3 which indicate presence of a hydroperoxide group (-OOH) in the fatty acid still appear even after 20 min from the initiation of the oxidation. These results reveal that superoxide dismutase (SOD) stabilize the hydroperoxide group (-OOH). Heretofore, disproportionation of a super oxide has been considered to be main activity of superoxide dismutase (SOD). From the above results, however, it is proved that superoxide dismutase (SOD) directly influence oxidized lipid.

Change with time in Oxidation of Arachidonic Acid with Soybean Lipoxygenase

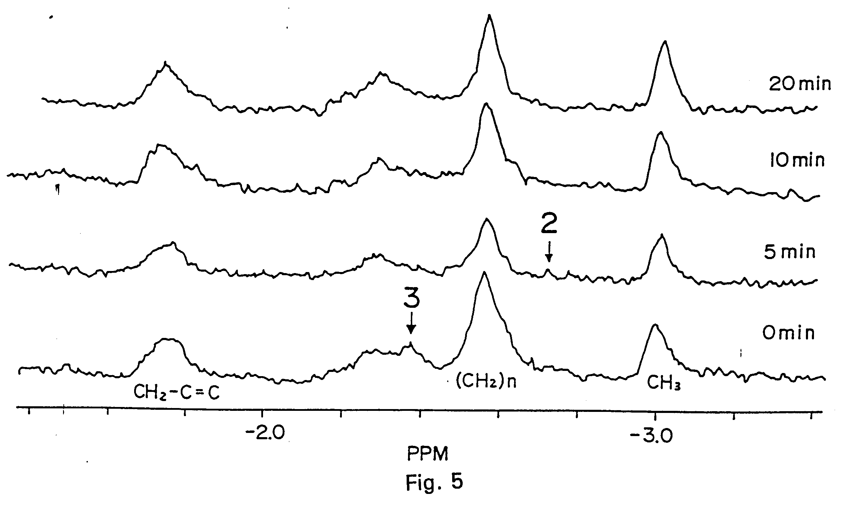

On the other hand, arachidonic acid is oxidized with soybean lipoxygenase in the same manner as in the oxidation of linoleic acid, and then measured by $^1$H-NMR spectroscopy (see Fig. 5).

The periods of time (min) shown in Fig. 5 indicate those for reaction with soybean lipoxygenase. In contrast to the case of linoleic acid, peaks attributable to arachidonic acid have already appeared under influence of dysprosium cation ($Dy^{3+}$) before oxidation with soybean lipoxygenase. This indicates that some of arachidonic acid has already been oxidized. This is considered to be attributable to the fact that arachidonic acid has already been auto-oxidized by oxygen in air. The oxidation patterns are also different from those of linoleic acid, and there is observed no clear peak which is considered to be influenced by hydroperoxide group (-OOH).

Change with Time in Oxidation of Arachidonic Acid with Soybean Lipoxygenase in the Presence of Superoxide Dismutase (SOD)

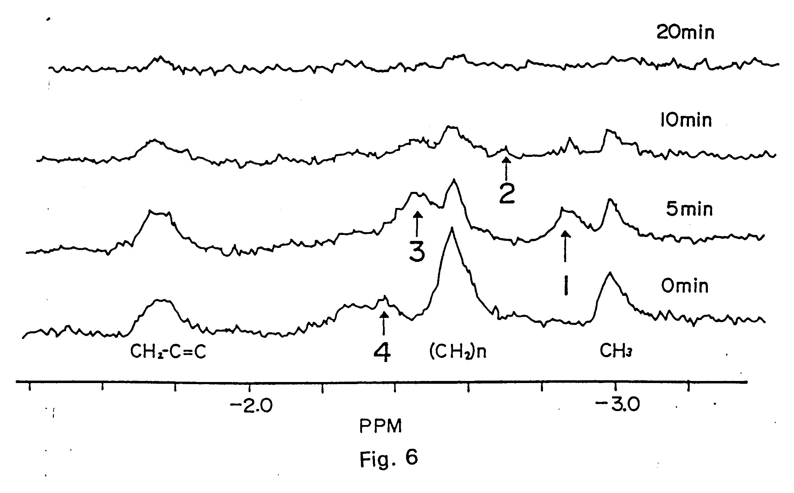

Arachidonic acid is oxidized with soybean lipoxygenase in the same conditions as above except that the oxidation is conducted in the presence of 0.1 U/ml superoxide dismutase (SOD). The samples are measured with time by $^1$H-NMR spectroscopy (see Fig. 6).

Peak 1 in Fig. 6 is clearly observed at 5 min after the initiation of oxidation, as compared with the peak which is considered to be influenced by a hydroperoxide group (-OOH) in the case where superoxide dismutase (SOD) is absent. Based also on this result, it is considered that superoxide dismutase (SOD) stabilizes a hydroperoxide group (-OOH). Further, it is considered that the presence of superoxide dismutase (SOD) facilitates decomposition of $CH_2$ and $CH_3$ which

constitute a skeleton of a fatty acid during oxidation step. This is proved based on the fact proton peaks ascribed to $CH_2$ and $CH_3$ substantially disappear by oxidation for a period of 15 min.

Formation of Oxidized Lipid

The present invention further preferably includes a process during which a water-soluble oxidized lipid is obtainable by:

adding superoxide dismutase (SOD) and $CuSO_4$ to a linoleic acid or arachidonic acid emulsion prepared by dissolving linoleic acid or arachidonic acid in deuterated methyl alcohol and adding the solution to a deuterated phosphate buffer under stirring, or to a low density lipoprotein solution sufficiently dialyzed against an undeuterated phosphate buffer; and

irradiating the resulting mixture with a long-wave ultraviolet light.

In this process, a linoleic acid emulsion, an arachidonic acid emulsion or a low density lipoprotein solution is used as a lipid material.

The linoleic acid emulsion is prepared by dissolving linoleic acid in deuterated methyl alcohol at a concentration of 0.1M, and adding the mixture to 20mM deuterated phosphate buffer under stirring. Specifically, 4mM linoleic acid emulsion is prepared.

On the other hand, low density lipoprotein is separated from human plasma by ultracentrifugation. This low density lipoprotein is sufficiently dialyzed against a 20mM undeuterated phosphate buffer under nitrogen-saturated atmosphere. The low density lipoprotein is then adjusted with 20mM phosphate buffer (pH 7.0) in Lowry method to such a volume that protein concentration is 1mg/ml.

0.1 U/ml superoxide dismutase (SOD) and $5\mu M$ $CuSO_4$ are preliminarily added to each of lipid samples prior to irradiation of the samples with ultraviolet light.

The thus prepared lipid samples are transferred to a vessel made of borosilicate, quartz or the like. Then, the samples are irradiated with long-wave ultraviolet light having the highest point of energy intensity at 365nm for various periods of time to form oxidized lipids.

This is considered to be attributable to the following fact. Unpaired electrons of $Cu^{2+}$ are excited by the long-wave ultraviolet light, and the electrons provide carbons having a double bond in the unsaturated fatty acid with electrons, thereby abstracting hydrogen atoms from the carbon atoms. Then oxidation is considered to take place in such a manner that an oxygen atom is bonded to the site where the abstraction has been occurred.

In order to form an oxidized lipid from a sample containing $Cu^{2+}$, long-wave ultraviolet rays having the highest point of energy intensity about 365nm are optimum. With irradiation of such a sample with short-wave ultraviolet rays having the highest point of energy intensity around 254nm, it is impossible to form a peroxide lipid containing a hydroperoxide group.

Further, it is also possible to produce an oxidized lipid containing a hydroperoxide group by irradiation of $Fe^{3+}$ or the like with ultraviolet light. Moreover, oxidation of arachidonic acid in the above-mentioned method also enables a water-soluble oxidized lipid containing a hydroperoxide group,which is believed to have considerable influence on a living body, to be produced.

Method of adding Dysprosium Tripolyphosphate

To the thus formed oxidized lipid is added a dysprosium tripolyphosphate as a lanthanide shift reagent.

200mM dysprosium chloride ($DyCl_3$) and 480mM pentasodium tripolyphosphate are thoroughly stirred in deuterium oxide. The solution, though it has a sediment, is adjusted to a pH 7.4 with deuterium chloride, thereby some of the sediment will be dissolved. Then, the solution is centrifuged at 3000g for 30 min to remove the remaining sediment. This dysprosium tripolyphosphate anion ($Dy(ppp)_2^{-7}$) solution is rapidly added to the oxidized samples at a concentration of 40mM.

Determination of Oxidized Lipid by $^1$H-NMR Spectroscopy

For the spectroscopy, hydrogen nucleus (proton) NMR analysis ($^1$H-NMR spectroscopy) by means of a nuclear magnetic resonance spectrometer is used.

NMR is conducted using a nuclear magnetic resonance spectrometer of 360 MHz. Proton spectra are obtained in quadrature detection mode at a probe temperature of 308K. The spectra are accumulated by repeating Free Induction Decay 512 times for linoleic acid samples and 32 times for low density lipoprotein samples. HDO and $H_2O$ proton signals are presaturated by 4 sec of 200mW of gated, continuous wave, single frequency irradiation. A spectral width of 7246 Hz and acquisition time of 0.565 sec are selected.

Results of determination

Oxidation patterns of linoleic acid using $Cu^{2+}$ and irradiation with ultraviolet light are very similar to those using soybean lipoxygenase, with respect to proton NMR spectra.

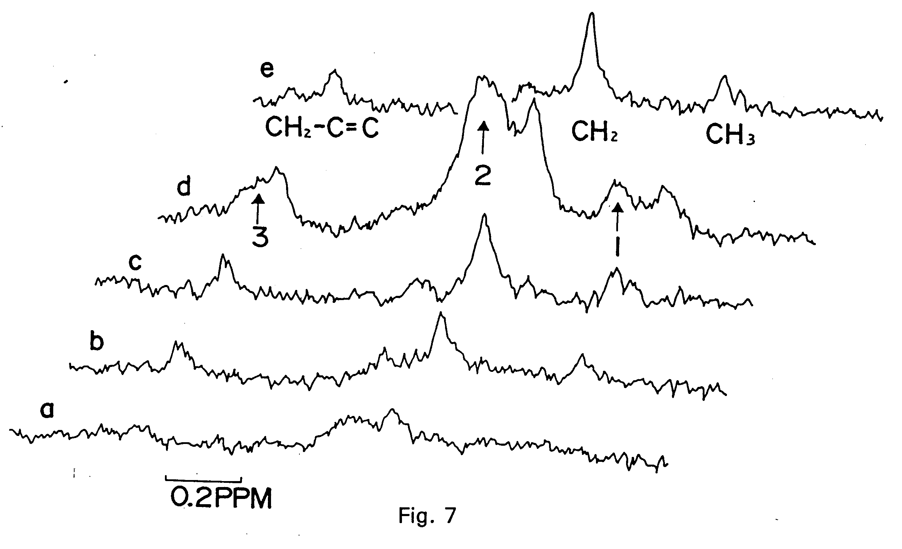

Fig. 7 shows results of measurement with time of oxidized samples by means of a proton nuclear magnetic resonance spectrometer, the oxidized samples having been prepared by preliminarily adding superoxide dismutase (SOD) and 5μM CuSO₄ to the linoleic acid samples already prepared in the above-mentioned manner, followed by irradiation with long-wave ultraviolet light. Pattern b in Fig. 7 is spectrum of the sample subjected to irradiation with long-wave ultraviolet light for 5 min. Patterns c, d, and e are spectra of the samples subjected to irradiation with long-wave ultraviolet light for 10, 20, and 30 min, respectively. Incidentally, pattern a is spectrum of the sample subjected no irradiation with long-wave ultraviolet light.

Peak 1 in Fig. 7 is apparently understood to be a proton signal influenced by a hydroperoxide group (-OOH). Peaks 2 and 3 are considered to be proton signals each influenced by a hydroperoxide group (-OOH) and another oxidative group. However, the oxygenated groups have not yet been confirmed what oxygenated groups they are.

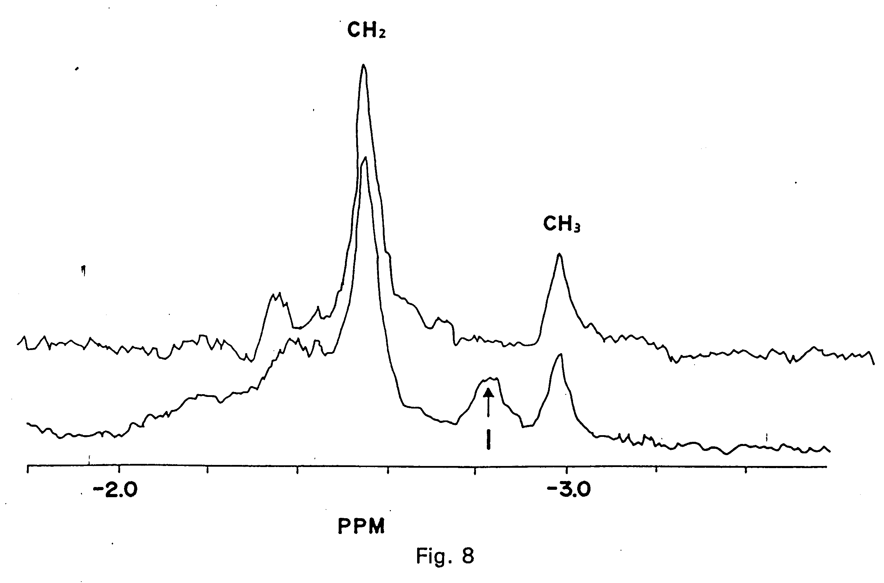

Fig. 8 shows the results of measurement with time by means of a proton nuclear magnetic spectrometer with respect to the linoleic acid samples oxidized by irradiation with long-wave ultraviolet light. The lower spectrum is derived from an oxidized lipid sample to which nothing has been added and which has been allowed to react for 10 min prior to the measurement. The upper spectrum is derived from an oxidized lipid sample to which 50nM glutathione and 0.2 U/ml (final concentration) glutathione peroxidase have been added and which has been allowed to react for 10 min prior to the measurement.

In the upper spectrum, peak 1 disappears under the influence of glutathione. From this, peak 1 is confirmed to be a proton peak influenced by a hydroperoxide group. This is based on the ground that glutathione peroxidase has been proved to convert its hydroperoxide group to a hydroxide group in the presence of glutathione.

It may be considered that the linoleic acid in the samples is a water-soluble linoleic acid containing both a hydroperoxide group having considerable influence on a living body and a polar oxidative group such as an epoxy group and an endoperoxy group. The reason is that an oxidized lipid containing only a hydroperoxide group is hardly water-soluble but is rather soluble in an organic solvent such as ethyl ether. However, with respect to oxidized linoleic acid, it might be water-soluble even if it contains only a hydroperoxide group. The hydroperoxide group can be stabilized by the presence of superoxide dismutase (SOD).

Formation of Oxidized Low Density Lipoprotein by the Use of Cu²⁺ and Irradiation with UV

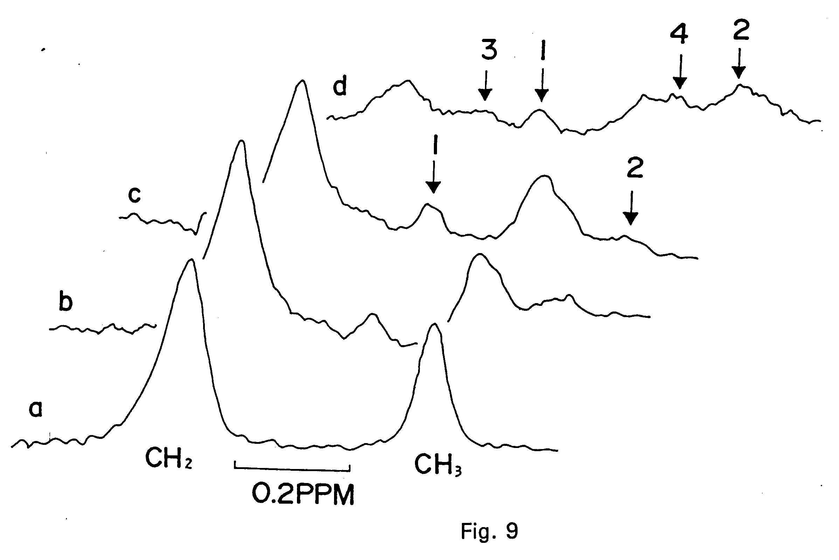

Fig. 9 shows results of measurement of samples in the following procedure. superoxide dismutase (SOD) and 5μM CuSO₄ are added to a low density lipoprotein solution, then the mixture is irradiated with long-wave ultraviolet light to oxidize the low density lipoprotein, and a dysprosium tripolyphosphate anion solution is added thereto, followed by measurement of the resultant samples by means of a proton nuclear magnetic resonance spectrometer. Pattern b in Fig. 9 is derived from the sample irradiated with long-wave ultraviolet light for 30 min. Patterns c and d are derived from the samples irradiated with long-wave ultraviolet light for 60 min and 90 min, respectively. Incidentally, pattern a is derived from the sample subjected no irradiation with long-wave ultraviolet light.

CH₂ and CH₃ in this Figure indicate proton signals corresponding to CH₂ and CH₃ in the skeleton of the fatty acid in the low density lipoprotein solution, respectively.

Peak 1 and peak 2 are separated from the peaks attributable to CH₂ and CH₃ due to the addition of the shift reagent, respectively. These peaks 1 and 2 are proton signals influenced by a cholesterol hydroperoxide group esterified with the fatty acid. The reason is that, of lipids present in the low density lipoprotein solution, no lipid other than cholesterol ester-bonded to the fatty acid can occupy the great peak as shown in the Figure in view of the quantity.

In other words, when these peaks are observed, it is recognized that a cholesterol hydroperoxide group is present in samples.

Stabilization of Hydroperoxide Group in Lipid by Acidification of Solution or by Addition of Ethylenediaminetetraacetic Acid (EDTA)

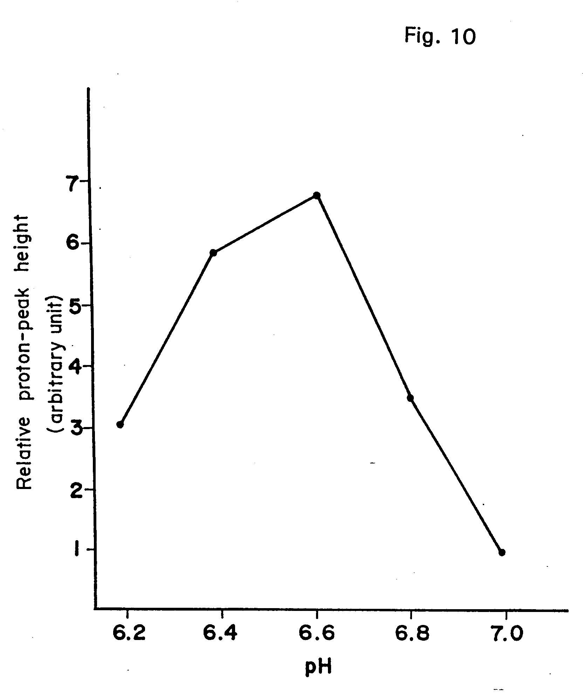

Fig. 10 is a trace showing the results of rough quantitative determination by measuring the heights of peak 1 influenced by a hydroperoxide group in low density lipoprotein. The quantity of hydroperoxide cholesterol in the low density lipoprotein reaches the peak at pH 6.6. It is considered that a hydroperoxide group is stabilized even under acidities lower than pH 6.6. Since a protein in the form of a lipoprotein such as a low density lipoprotein undergoes degeneration under acidity of pH 6 or lower, the quantity of the hydroperoxide group is apparently measured such that it becomes lowered under acidity of pH 6 or lower. When linoleic acid is used, a hydroperoxide group in linoleic acid is stabilized even under strong acidity of pH 3 to pH 4. On the other hand, addition of a chelating agent such as EDTA is proved to be capable of stabilizing the above-mentioned hydroperoxide group even under neutrality in the vicinity of pH 7.0. Instead of EDTA dysprosium tripolyphosphate may also be used These phenomena indicate that acidification of an oxidized lipid or addition of a chelating agent enables the oxidized lipid samples prepared in the manner as described above to be preserved.

Comparison Between Thiobarbituric Acid Reaction Substance Measuring Method and $^1$H-NMR Spectroscopy by the Use of Oxidized Low Density Lipoprotein

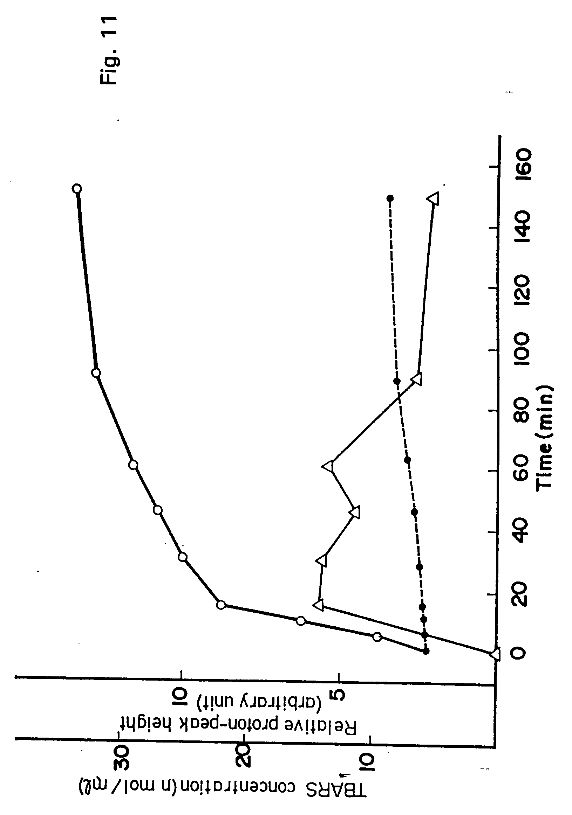

Fig. 11 shows the results of measurements of change with time of oxidation of the low density lipoprotein irradiated with long-wave ultraviolet light by thiobarbituric acid reactive substance measuring method which is conventionally widely employed (white circles in Fig. 11) and by $^1$H-NMR spectroscopy (white triangles in Fig. 11). Incidentally, a sample is taken as a control in advance of the irradiation with long-wave ultraviolet light, and allowed to stand in a dark place. Change of oxidation of the low density lipoprotein with respect to the control sample is also measured with time by TBARS measuring method. The results are shown by black circles in Fig. 11.

In thiobarbituric acid reactive substance measuring method, a thiobarbituric acid reactive substance which reflects accumulative oxidation degree rapidly increases from immediately after the irradiation with long-wave ultraviolet light, and the increase rate thereof becomes moderate 30 min later. The height of peak 1 reaches the highest value 20 min after the initiation of the irradiation. Thereafter, however, the height of peak 1 becomes lower, although the value for the thiobarbituric acid reactive substance continues to increase even after elapse of time of 60 min. There is observed disparity between them. The thiobarbituric acid reactive substance is measured in terms of the products decomposed through oxidation of a lipid, such as methyl malonaldehyde. Accordingly, the value represents the accumulation of the products.

On the other hand, in the $^1$H-NMR spectroscopy method according to the present invention, the water-soluble oxidized lipid having a hydroperoxide group per se is measured. Accordingly, as disappearance of the unstable hydroperoxide group occurs, the height of the peak 1 decreases directly reflecting the disappearance of the hydroperoxide group.

As described above, although the conventional thiobarbituric acid reaction substance measuring method can detect occurrence of oxidation of a lipid, the method is not capable of proving the existence of a certain kind of an oxidized lipid in a sample.

The present invention detects and determines a specimen to include a water-soluble oxidized lipid through analyzing the difference in proton signals by spectroscopy which is observed, when dysprosium tripolyphosphte anion $(Dy(ppp)_2^{-7})$ is added to the specimen, between the case where the sample contains a water-soluble oxidized lipid and the case where the sample does not contain a water-soluble oxidized lipid. Therefore, a specimen is readily and accurately determined to contain a water-soluble oxidized lipid.

In other words, according to the present invention, a specimen can directly be detected and determined to contain a water-soluble oxidized lipid through analyzing the difference in proton signals by spectroscopy.

Further, even if an impurity is contained in a specimen, the specimen can be detected and determined to contain an oxidized lipid without influence of the presence of the impurity.

Moreover, the determination process is extremely simple by virtue of the adoption of the spectroscopic proton signal analysis of the specimen, and the specimen can be detected and determined to contain an oxidized lipid in a short period of time. In addition, since no treatment of the specimen such as esterification is required at all, there is no longer any undesired possibility of denaturation of the specimen.

On the other hand, by addition of a lanthanide shift reagent such as dysprosium cation $(Dy^{3+})$ to an oxidized lipid and subsequent $^1$H-NMR spectroscopic analysis, it has for the first time been rendered possible to confirm the presence of the hydroxide group (-OH) in the oxidized lipid.

Further, it is successfully attained by the present invention to form a water-soluble oxidized lipid containing an unstable hydroperoxide group, which is considered to have marked influence on a living body.

In addition, there is found cumulative advantageous effect attendant upon the formation of the water-soluble oxidized lipid having a hydroperoxide group by the use of $Cu^{2+}$ and SOD and irradiation with ultraviolet light, and on the basis of the finding, it is successfully accomplished to detect and determine the water-soluble oxidized lipid containing a hydroperoxide group by $^1$H-NMR spectroscopic analysis.

Still further, it is rendered possible to detect presence of an oxidized cholesterol-fatty acid ester contained in low density lipoprotein at a concentration substantially the same as or lower than that in human plasma.

From the above effects, the proton-NMR spectroscopic analysis by means of a nuclear magnetic resonance spectrometer is considered to be effective as a process which is capable of directly detecting presence of an oxidized lipid in a biomaterial containing many impurities such as plasma.

There are disclosed a process for detecting and determining an oxidized lipid, which is capable of readily and accurately determining a specimen to contain a water-soluble oxidized lipid, and a process during which a water-soluble oxidized lipid is formed having a hydroperoxide group which has specific influence on a living body. A specimen is detected and determined to contain a water-soluble oxidized lipid by adding a lanthanide shift reagent to a specimen, followed by spectroscopic analysis thereof. An oxidized lipid is formed e.g. by adding superoxide dismutase (SOD) and $CuSO_4$ to a linoleic acid or arachidonic acid emulsion prepared by dissolving linoleic acid or arachidonic acid in deuterated methyl alcohol and adding the solution to a deuterated phosphate buffer under stirring, or to a low density lipoprotein solution sufficiently dialyzed against an undeuterated phosphate buffer; followed by irradiation with long-wave ultraviolet light.

## Claims

1. A process for detecting and determining a water-soluble oxidized lipid in a specimen, said process comprising

   adding a lanthanide shift reagent to a specimen, and subjecting the resultant mixture to spectroscopy to detect and determine said water-soluble oxidized lipid.

2. The process according to claim 1, wherein the specimen is linoleic acid or arachidonic acid oxidized by soybean lipoxygenase.

3. The process according to claim 1 or 2, wherein the lanthanide shift reagent is dysprosium tripolyphosphate anion.

4. The process according to claim 1, wherein the said water-soluble oxidized lipid is a lipid obtainable by

   adding superoxide dismutase (SOD) and $CuSO_4$ to (i) a linoleic acid or arachidonic acid emulsion prepared by dissolving linoleic acid or arachidonic acid in deuterated methyl alcohol and adding the solution to a deuterated phosphate buffer under stirring, or to (ii) a low density lipoprotein solution sufficiently dialyzed against an undeuterated phosphate buffer; and the resulting mixture is irradiated with a long-wave ultraviolet light to form said water-soluble oxidized lipid.

5. The process according to claim 1, wherein said water-soluble oxidized lipid is a lipid obtainable by

   adding a dysprosium tripolyphosphate anion to the irradiated mixture prepared according to claim 4 as said lanthanide shift reagent and said water-soluble oxidized lipid is detected by proton-NMR spectroscopy by means of a nuclear magnetic resonance spectrometer.

6. The process according to claim 1 or 4, wherein said water-soluble oxidized lipid is a water-soluble oxidized lipid having a hydroperoxide group.

7. The process according to claim 6, further comprising stabilizing said water-soluble oxidized lipid by maintaining said water-soluble oxidized lipid under acidic conditions at a pH in the range of 3 to 4 or adding to said water-soluble oxidized lipid a chelating agent selected from the group consisting of dys-prosium tripolyphosphate and ethylene-diaminetetraacetic acid (ETDA).

8. The process according to claim 1, wherein said spectroscopy is $^{13}$C-NMR spectroscopy or $^1$H-NMR spectroscopy.

## Patentansprüche

1. Verfahren zum Nachweis und zur Bestimmung von wasserlöslichem oxidierten Lipid in einer Probe, wobei das Verfahren die Zugabe eines Lanthanid-Verschiebungsreagenzes zu einer Probe und das Unterziehen der resultierenden Mischung einer spektroskopischen Analyse umfaßt, um das wasserlösliche oxidierte Lipid nachzuweisen und zu ermitteln.

2. Verfahren nach Anspruch 1, in dem die Probe aus Linolsäure oder Arachidonsäure besteht, die mittels Sojabohnen-Lipoxygenase oxidiert wurden.

3. Verfahren nach Anspruch 1 oder Anspruch 2, in dem das Lanthanid-Verschiebungsreagens aus Dysprosiumtripolyphosphat-Anionen besteht.

4. Verfahren nach Anspruch 1, in dem das wasserlösliche oxidierte Lipid ein Lipid ist, das durch die Zugabe von Superoxid-Dismutase (SOD) und $CuSO_4$ zu (i) einer Linolsäure- oder Arachidonsäure-Emulsion, die durch Lösen von Linolsäure oder Arachidonsäure in deuteriertem Methylalkohol und die Zugabe der Lösung zu einem deuterierten Phosphatpuffer unter Rühren hergestellt wurde, oder zu (ii) einer Lösung eines Lipoproteins geringer Dichte, die ausreichend gegenüber einem nicht-deuterierten Phosphatpuffer dialysiert wird; und Bestrahlen der resultierenden Mischung mit langwelligem ultravioletten Licht, um das wasserlösliche oxidierte Lipid zu erzeugen, erhältlich ist.

5. Verfahren nach Anspruch 1, in dem das wasserlösliche oxidierte Lipid ein Lipid ist, das durch die Zugabe von Dysprosiumtripolyphosphat-Anionen als Lanthanid-Verschiebungsreagens zu der bestrahlten Mischung, die nach Anspruch 4 hergestellt wurde, erhältlich ist, und das wasserlösliche oxidierte Lipid durch Protonen-NMR-Spektroskopie mittels eines Kernresonanzspektrometers nachgewiesen wird.

6. Verfahren nach Anspruch 1 oder Anspruch 4, in dem das wasserlösliche oxidierte Lipid ein wasserlösliches oxidiertes Lipid mit einer Hydroperoxidgruppe ist.

7. Verfahren nach Anspruch 6, das desweiteren die Stabilisierung des wasserlöslichen oxidierten Lipids durch Halten des wasserlöslichen oxidierten Lipids unter sauren Bedingungen bei einem pH-Wert in einem Bereich von 3 bis 4, oder die Zugabe einer chelatbildenden Substanz, die aus der Gruppe ausgewählt ist, die aus Dysprosiumtripolyphosphat und Ethylendiamintetraessigsäure (EDTA) besteht, zu dem wasserlöslichen oxidierten Lipid umfaßt.

**8.** Verfahren nach Anspruch 1, in dem die spektrosko-pische Analyse aus einer $^{13}$C-NMR-Spektroskopie oder einer $^{1}$H-NMR-Spektroskopie besteht.

**Revendications**

**1.** Procédé pour détecter et déterminer un lipide oxydé hydrosoluble dans un échantillon, ledit procédé comprenant l'addition d'un réactif de déplacement de type lanthanide à un échantillon et la soumission du mélange résultant à une spectroscopie pour détecter et déterminer ledit lipide oxydé hydrosoluble.

**2.** Procédé selon la revendication 1, dans lequel l'échantillon est l'acide linoléique ou l'acide arachidonique oxydé par la lipoxygénase de soja.

**3.** Procédé selon la revendication 1 ou 2, dans lequel le réactif de déplacement de type lanthanide est un anion tripolyphosphate de dysprosium.

**4.** Procédé selon la revendication 1, dans lequel ledit lipide oxydé hydrosoluble est un lipide qui peut être obtenu par addition de superoxyde dismutase (SOD) et de $CuSO_4$ à (i) une émulsion d'acide linoléique ou d'acide arachidonique préparée par dissolution d'acide linoléique ou d'acide arachidonique dans l'alcool méthylique deutéré et addition de la solution à un tampon phosphate deutéré sous agitation, ou à (ii) une solution lipoprotéique à basse densité suffisamment dialysée contre un tampon phosphate non deutéré ; et le mélange résultant est irradié avec une lumière ultraviolette de grande longueur d'onde pour former ledit lipide oxydé hydrosoluble.

**5.** Procédé selon la revendication 1, dans lequel ledit lipide oxydé hydrosoluble est un lipide qui peut être obtenu par addition d'un anion tripolyphosphate de dysprosium à titre dudit réactif de déplacement de type lanthanide au mélange irradié préparé selon la revendication 4, et ledit lipide oxydé hydrosoluble est détecté par spectroscopie RMN du proton au moyen d'un spectromètre de résonance magnétique nucléaire.

**6.** Procédé selon la revendication 1 ou 4, dans lequel ledit lipide oxydé hydrosoluble est un lipide oxydé hydrosoluble ayant un groupe hydroperoxyde.

**7.** Procédé selon la revendication 6 comprenant en outre la stabilisation dudit lipide oxydé hydrosoluble par maintien dudit lipide oxydé hydrosoluble dans des conditions acides à un pH dans le domaine de 3 à 4 ou addition audit lipide oxydé hydrosoluble d'un agent chélatant choisi dans le groupe consistant en le tripolyphosphate de dysprosium et l'acide éthylènediaminetétraacétique (EDTA).

**8.** Procédé selon la revendication 1, dans lequel ladite spectroscopie est la spectroscopie RMN de $^{13}$C ou la spectroscopie RMN de $^{1}$H.

Fig. 1

EP 0 631 138 B1

Fig. 2

EP 0 631 138 B1

Fig. 3

EP 0 631 138 B1

EP 0 631 138 B1

CH₂-C=C

(CH₂)n

CH₃

30min

20min

10min

5min

5 6

4 3

1

0min

−2.0

−3.0

PPM

Fig. 4

CH$_2$-C=C    (CH$_2$)n    CH$_3$

20 min

10 min

5 min

0 min

2

3

-2.0    -3.0

PPM

Fig. 5

EP 0 631 138 B1

16

Fig. 6

Fig. 7

0.2PPM

Fig. 8

Fig. 9

Fig. 10

Fig. 11

EP 0 631 138 B1